# FASCICULE DE BREVET EUROPEEN

(11) **EP 0 986 350 B1**
(45) Date de publication et mention de la délivrance du brevet: **02.01.2003**
(21) Numéro de dépôt: 98922911.7
(22) Date de dépôt: 30.04.1998
(51) Int. Cl.: A61F 2/44

(54) **IMPLANT NOTAMMENT POUR LE REMPLACEMENT D'UN CORPS VERTEBRAL EN CHIRURGIE DU RACHIS**
IMPLANTAT, INSBESONDERE ZUM EINSATZ EINES WIRBELKÖRPERS IN DER WIRBELSÄULENCHIRURGIE
IMPLANT IN PARTICULAR FOR REPLACING A BODY OF VERTEBRAE IN BACKBONE SURGERY

(30) Priorité: 02.05.1997 FR 9705465
(43) Date de publication de la demande: 22.03.2000
(73) Titulaire: Stryker France S.A., 93290 Tremblay-en-France (FR)
(72) Inventeur: CROZET, Yves, F-74600 Seynod (FR); MANGIONE, Paolo, F-33600 Pessac (FR)
(74) Mandataire: Le Forestier, Eric
(86) Numéro de dépôt international: FR9800879
(87) Numéro de publication internationale: WO98049975

(56) Documents cités:
- WO-A-97/00054
- FR-A- 2 636 227
- US-A- 5 192 327
- US-A- 5 290 312
- US-A- 5 522 899

## Description

La présente invention a trait d'une façon générale aux implants pour la chirurgie du rachis, et concerne plus particulièrement un nouvel implant destiné à remplacer un corps vertébral atteint notamment d'une tumeur ou d'un traumatisme. Un implant selon les caractéristiques du préambule de la revendication 1 est connu du document US-A-5 522 899.

On connaît déjà un certain nombre d'implants, qui comprennent classiquement deux éléments d'appui sur les plateaux vertébraux sus-jacent et sous-jacent, et un moyen pour faire varier la distance entre ces éléments d'appui et ainsi ajuster l'implant à la hauteur requise en fonction de la morphologie rachidienne du patient.

Le moyen pour faire varier la hauteur de l'implant peut comprendre un organe tournant, et des moyens du genre vis-écrou.

Un inconvénient majeur de cet implant connu réside en ce que l'organe tournant doit être déplacé autour d'un axe sensiblement parallèle à l'axe de la colonne vertébrale. Ceci s'avère extrêmement fasdidieux pour amener l'implant à la hauteur voulue, car l'outil utilisé pour faire tourner cet organe ne peut faire, compte-tenu de l'environnement du patient, qu'une fraction réduite d'un tour complet, et doit alors être manoeuvré de nombreuses fois pour atteindre la hauteur voulue. Ceci d'autant plus que le pas du filetage est impérativement faible pour engendrer l'effort axial nécessaire à la distraction, sans toutefois risquer de produire un glissement entre les éléments d'appui et les plateaux vertébraux sus- et sous-jacents.

La présente invention vise principalement à pallier cet inconvénient, et à proposer un implant de remplacement de corps vertébral qui soit plus simple à manier pour sa mise en place et pour opérer la distraction, et qui en outre soit doté d'une excellente stabilité face aux efforts de compression axiaux auxquels il sera exposé.

Ainsi la présente invention propose un implant notamment pour le remplacement d'un corps vertébral en chirurgie du rachis, comprenant des premiers et second éléments d'appui contre des plateaux vertébraux sus- et sous-jacents, des moyens pour retenir les deux éléments d'appui l'un au-dessus de l'autre et au moins un organe mobile apte à faire varier la distance entre les deux éléments d'appui, les moyens de retenue comprenant au moins un moyen à glissière prévus entre les deux éléments d'appui, caractérisé en ce que le ou chaque organe mobile est constitué par une came à positions discrètes apte à être entraînée en rotation autour d'un axe essentiellement horizontal et essentiellement parallèle au plan sagittal.

Des aspects préférés, mais non limitatifs, de l'implant selon l'invention sont les suivants :
- la ou chaque came à positions discrètes présente en section transversale un contour en forme de polygone irrégulier.
- la ou chaque came possède une pluralité de paires de faces, les faces d'une même paire étant mutuellement parallèles, et chaque paire de faces possédant une distance entre faces différente de celle des autres paires.
- la came possède trois paires de faces.
- chaque élément d'appui possède un plateau d'appui sur une face duquel est ménagée une rainure, et au moins certaines paires de faces sont dimensionnées de manière à présenter une longueur, selon la circonférence de la came, très légèrement inférieure à la largeur de chaque rainure.
- les faces d'au moins une paire de faces de la came présentent une longueur, selon 1a circonférence de la came, qui est ajustée à la largeur des rainures des plateaux d'appui à l'aide d'un décrochement prévu au niveau de sa transition avec une face voisine.
- la ou chaque came est montée à rotation et à translation sur le ou chaque moyen à glissière.
- le ou chaque moyen à glissière comprend un montant creux formant glissière formé à partir d'un élément de l'implant et un élément formant coulisseau formé à partir d'un autre élément de l'implant, et la glissière et le coulisseau sont traversés par des ouvertures allongées pour le passage d'un axe de montage de la came.
- l'axe de montage de la came est défini par une vis traversant un passage central de la came et lesdites ouvertures allongées, et engagée dans un écrou.
- l'une des ouvertures allongées de la glissière est une encoche apte à assurer le blocage de l'écrou contre sa rotation.
- les éléments d'appui possèdent à leur surface extérieure des dents d'ancrage dans des vertèbres sus- et sous-jacente à l'implant.
- l'implant comprend deux éléments d'appui et un élément central, deux cames opérant respectivement encre l'élément d'appui supérieur et l'élément central et entre l'élément d'appui inférieur et l'élément central, et deux moyens formant glissières opérant respectivement entre l'élément d'appui supérieur et l'élément central et entre l'élément d'appui inférieur et l'élément central.

D'autres aspects, buts et avantages de la présente invention apparaîtront mieux à la lecture de la description détaillée suivante d'une forme de réalisation préférée de celle-ci, donnée à titre d'exemple et faite en référence au dessin annexé, sur lequel :
les figures 1 et 2 sont des vues en perspective éclatée d'un implant selon l'invention, selon deux directions d'observation différentes,
les figures 3 et 4 sont des vues en perspective d'un implant selon l'invention à l'état assemblé, selon deux directions d'observation différentes,
la figure 5 est une vue en élévation de côté de l'implant assemblé des figures 3 et 4,
la figure 6 est une vue en élévation de face de l'implant assemblé des figures 3 à 5,
la figure 7 est une vue de dessus de l'implant assemblé des figures 3 à 6, et
la figure 8 est une vue en perspective éclatée d'une variante de réalisation de l'implant.

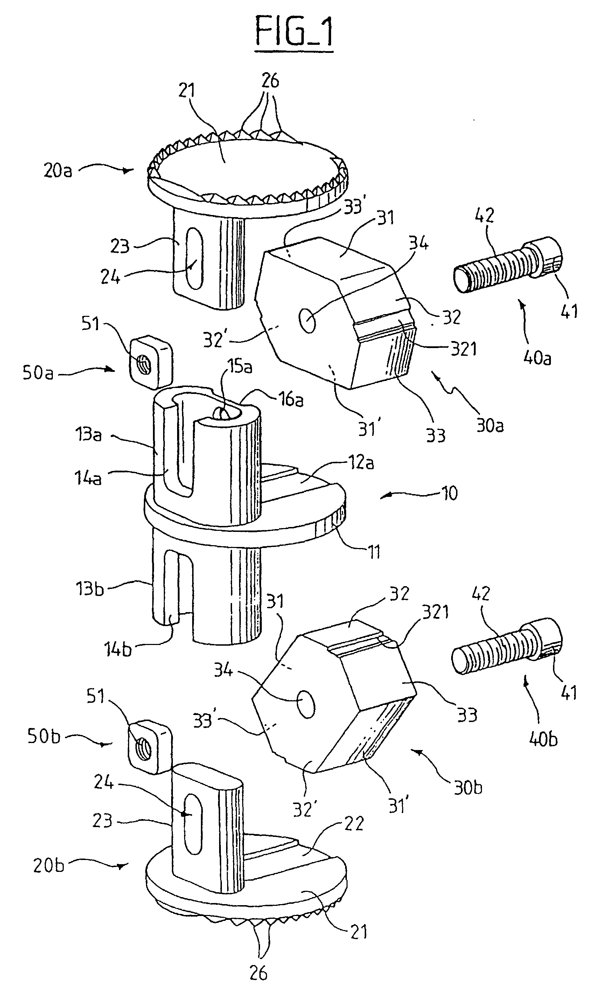

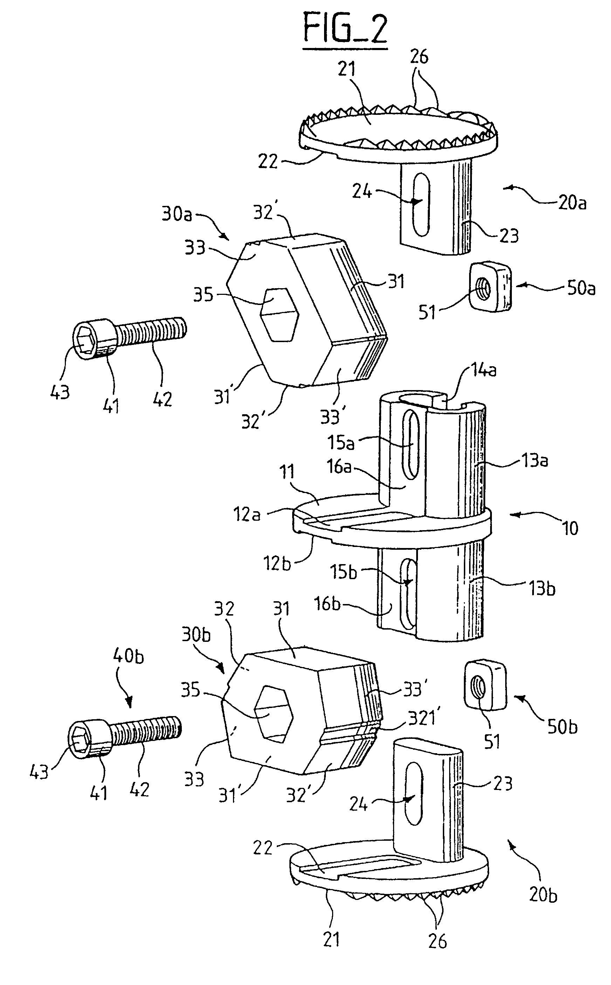

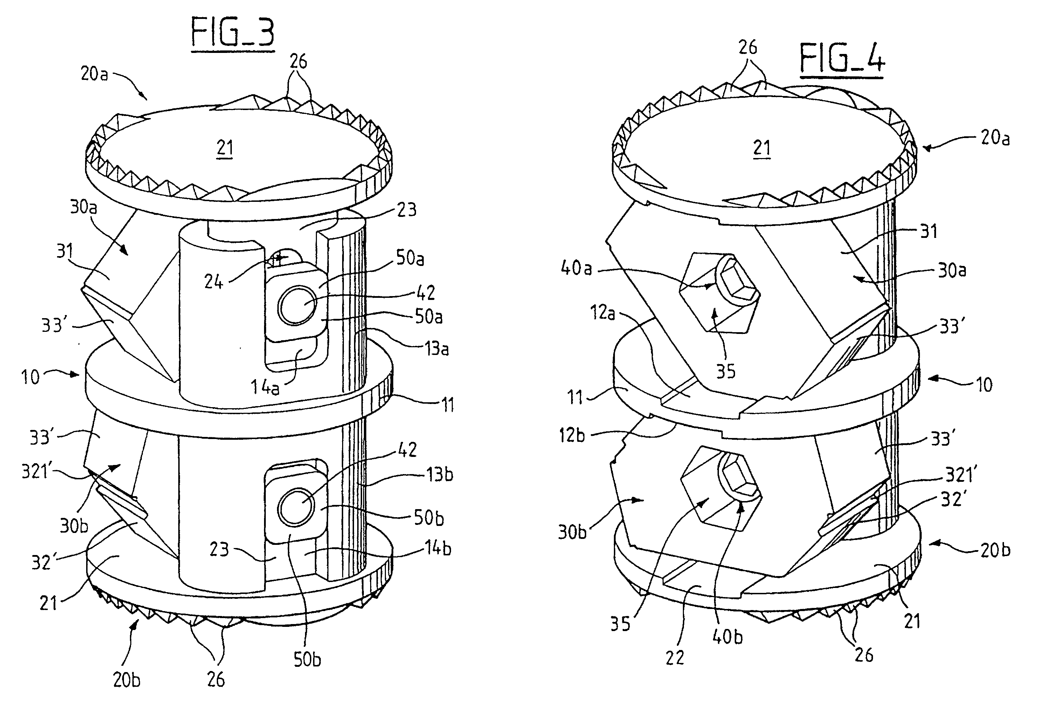

En référence aux figures 1 à 7, on a représenté un implant de remplacement d'un corps vertébral qui comprend sept éléments, à savoir un élément central 10, deux éléments d'appui 20a et 20b, respectivement supérieur et inférieure, deux éléments formant cames à positions discrètes, respectivement 30a et 30b, l'une opérant entre l'élément 10 et l'élément 20a, et l'autre opérant entre l'élément 10 et l'élément 20b, et enfin deux vis 40a, 40b aptes à coopérer avec deux écrous 50a, 50b pour maintenir fermement l'ensemble de l'implant dans la position requise.

L'élément central 10 comporte un plateau intermédiaire 11 en forme générale de disque horizontal. A partir de ce disque s'étendent vers le haut un premier montant cylindrique creux formant glissière 13a, et vers le bas un second montant cylindrique creux formant glissière 13b, situé dans le prolongement vertical du montant 13a. La section des glissières 13a, 13b est en forme d'ovale. Chaque glissière s'étend au voisinage d'un bord du disque 11 et comporte sur sa face intérieure une lumière traversante oblongue, respectivement 15a, 15b, et sur sa face externe une encoche allongée verticalement, respectivement 14a, 14b, légèrement plus large et débouchant sur l'extrémité libre de la glissière associée.

Par ailleurs, chaque glissière 13a, 13b comporte sur sa face interne un léger renfoncement, respectivement 16a, 16b, au fond duquel se trouve la lumière oblongue 15a, 15b.

Enfin l'élément central 10 comporte sur la face supérieure du disque 11 une rainure 12a de largeur prédéterminée et de profondeur réduite, s'étendant dans une direction essentiellement diamétrale du disque, à partir de la glissière 13a et jusqu'à son bord opposé. Dans 1a face inférieure du disque 11 est formé une rainure 12b identique et symétrique.

L'élément supérieur 20a de l'implant comporte un plateau supérieur 21 à partir duquel s'étend vers le bas, d'un seul tenant avec lui, un coulisseau plein 23 dont la section est légèrement plus petite que la section intérieure de la glissière 13a, ce coulisseau 23 étant traversé de part en part par une lumière 24 de taille voisine de celle de la lumière 15a de l'élément 10.

Sur la surface supérieure du plateau 21 sont formées une série de dents 26, notamment de forme pyramidale, à des fins expliquées plus loin. Enfin sur la surface inférieure du plateau 21 est formé une rainure 22 de forme et de taille préférentiellement identiques à celles de la rainure 12a du disque central 11 de l'élément 10.

L'élément inférieure 20b est en l'espèce rigoureusement identique à l'élément supérieur 20a, en étant simplement retourné à 180° sur lui-même.

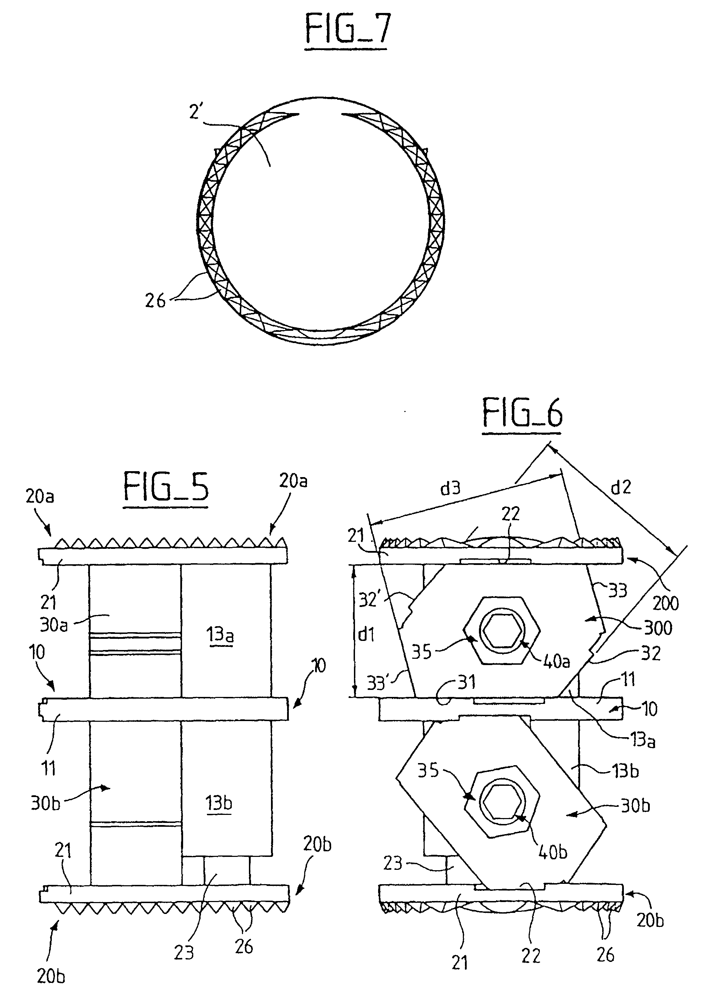

L'élément formant came à position discrètes destiné à intervenir entre les éléments 10 et 20a, désigné par la référence 30a, présente en section transversale un coutour généralement hexagonal irrégulier. Plus précisément. L'élément 30a possède :
- deux grandes faces 31, 31', opposées et parallèles l'une à l'autre et séparées par une distance orthogonale d1 (voir figure 6);
- deux faces de taille intermédiaire 32, 32', opposées et parallèles l'une à l'autre et séparées par une distance orthogonale d2 supérieure à d1 ;
- enfin deux petites faces 33, 33', opposées et parallèles l'une à l'autre et séparées par une distance orthogonale d3 supérieure à d2.

On observera en outre que la transition entre les faces 31 et 33 d'une part, et 31' et 33' d'autre part, est légèrement arrondie.

On observe également qu'à la transition entre les faces 32 et 33 d'une part, et 32' et 33' d'autre part, il est prévu un décrochement, respectivement 321, 321', dont le fond est parallèle à la face respective 32, 32' et dont la profondeur est sensiblement égale à celle des rainures 12a, 12b et 22 ménagées respectivement d'une part sur chaque face du disque central 11 de l'élément 10 et d'autre part sur la face intérieure des plateaux 21 des éléments 20a, 20b.

En outre, les longueurs (c'est-à-dire les dimensions dans le sens circonférentiel) des différentes faces de l'élément 30a sont comme suit :
- les grandes faces 31, 31' ont une longueur supérieure à la largeur des rainures 12a, 12b et 22;
- en choisissant de façon appropriée la largeur des décrochements 321, 321', les faces moyennes 32, 32' ont une longueur effective très légèrement inférieure à la largeur des rainures 12a, 12b et 22;
- enfin les petites faces 33, 33' ont également une longueur effective très légèrement inférieure à la largeur des rainures 12a, 12b et 22.

L'élément 30a possède par ailleurs un alésage central traversant central 34 qui se termine, au niveau de l'une des faces latérales de l'élément, par une partie élargie 35 de section hexagonale, formant une empreinte destinée à recevoir un outil comme on le verra plus loin.

L'élément formant came 30b destiné à opérer entre les éléments 10 et 20b est en l'espèce rigoureusement identique à l'élément 30a.

L'implant selon l'invention comporte enfin deux vis 40a et 40b, identiques et possédant chacune une tête 41 pourvue d'une empreinte 43 pour outil de vissage, et une tige filetée 42, ainsi que deux écrous 50a, 50b de contour carré à coins arrondis, traversés chacun par un alésage taraudé 51 complémentaire du filetage des vis.

On notera ici que :
- la section transversale des empreintes 35 est plus grande que celle des têtes 41 des vis 40a, 40b, tandis que la profondeur desdites empreintes 35 est sensiblement supérieure à la dimension axiale desdites têtes 41, 40b,
- le diamètre hors-tout des tiges filetées 42 est légèrement inférieur au diamètre des alésages 34 des éléments 30a, 30b,
- le côté de chaque écrou 50a, 50b est choisi très légèrement plus petit que la largeur des encoches 14a, 14b formées dans les montants 13a, 13b de l'élément 10.

On va maintenant décrire le montage de l'implant selon l'invention, puis les différentes étapes de la pose par le chirurgien.

Tout d'abord, l'implant est assemblé en introduisant les coulisseaux 23 des éléments 20a, 20b dans les glissières respectives 13a, 13b de l'élément 10. On prépositionne ensuite les éléments 30a, 30b d'une part entre le plateau 21 de l'élément 20a et le plateau 11, et d'autre part entre le le plateau 21 de l'élément 20b et le plateau 11, de telle sorce que leurs alésages 34 soient au droit des fentes respectives 15a, 15b. Puis les vis 40a, 40b sont engagées dans les passages respectifs 35, 34, dans les fentes 15a, 15b et dans les encoches 14a, 14b, où elles sont vissées dans les écrous 50a, 50b.

Avant la pose, les éléments formant cames 30a, 30b sont initialement réglés à leur hauteur minimale, c'est-à-dire que leurs grandes faces 31, 31' sont en appui contre les faces en vis-à-vis des plateaux respectifs, en débordant de part et d'autre des rainures 12a, 12b et 22 pour assurer un appui stable.

A ce stade, les vis 40a, 40b ne sont pas serrées, pour autoriser la rotation ultérieure des éléments 30a, 30b.

Dans cet état de hauteur minimale, l'implant est mis en place par le chirurgien entre les plateaux vertébraux des vertèbres sus- et sous-jacente, en opérant si nécessaire une légère distraction.

Une fois cette mise en place effectuée, on exerce sur les éléments 30a, 30b, à l'aide d'un outil engagé dans leurs empreintes 35, une rotation permettant d'amener chacun de ces éléments:
- soit dans une position intermédiaire, dans laquelle les surfaces 32, 32' sont en appui contre les plateaux respectifs, et plus précisément dans le fond des rainures 22 et 12a (ou 22 et 12b) ;
- soit dans une position de hauteur maximale, dans laquelle les surfaces 33, 33' sont en appui contre les plateaux respectifs, et plus précisément, ici encore, dans le fond des rainures 22 et 12a (ou 22 et 12b).

Cette rotation, du fait qu'un couple important peut être exercé de façon extrêmement aisée par accès postérieur sur les éléments 30a, 30b, permet d'opérer une distraction contrôlée entre les plateaux vertébraux sus- et sous-jacents. La stabilité de l'implant au cours de cette distraction est assurée par les dents 26 des plateaux supérieur et inférieur 21, qui mordent dans les plateaux vertébraux sus- et sous-jacents sous l'action de l'effort axial résultant d'action des cames.

On observera ici que la présence de deux éléments formant cames à trois positions discrètes permet d'obtenir une variété suffisante de hauteurs d'implant, données respectivement par les combinaisons de hauteurs de cames :
d1 et d1
d1 et d2
d1 et d3
d2 et d2
d2 et d3
d3 et d3.

On observera ici qu'avec deux éléments 30a, 30b de géométries différentes l'une de l'autre, le nombre de combinaisons peut encore être augmenté.

On observera par ailleurs que le calage des surfaces 32, 32' ou 33, 33' d'une came 30a ou 30b dans les rainures 12a et 22 ou 12b et 22 participe également à la stabilité de l'implant en garantissant une stablité de la position angulaire de la came, même en présence d'efforts de compression importants exercés, après distraction, par les vertèbres sus- et sous-jacentes.

La stabilité de la came sans sa position de moindre hauteur (cas où les surfaces 31 et 31' sont les surfaces d'appui) est garantie quant à elle par la longueur de ces surfaces.

Après avoir réglé l'implant à la hauteur voulue, les vis 40a, 40b sont serrées à l'aide d'un outil approprié pour confirmer la fixation des différents éléments dans la position requise, les écrous 50a, 50b étant bloqués contre toute rotation dans les encoches 14a, 14b.

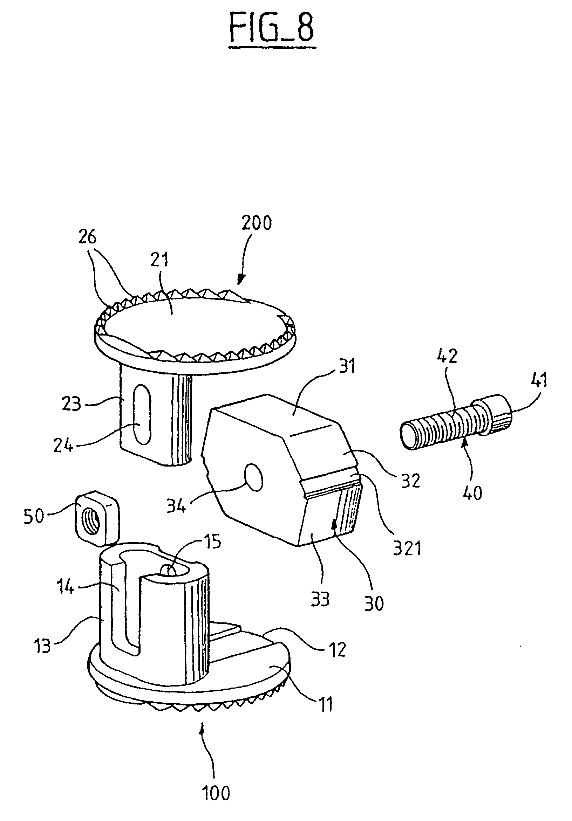

On a représenté sur la figure 8 une variante de réalisation de l'invention qui peut être utilisée lorsque l'on souhaite obtenir un implant de hauteur réduite.

Il diffère de celui des figures 1 à 7 par le fait qu'il est prévu un seul élément formant came 30, en association avec deux éléments d'appui vertébral 100 et 200. L'élément 100 comporte un plateau inférieur 11 muni de dents 26 sur sa face inférieure et d'une glissière 13 et d'une rainure 12 sur sa face supérieure. L'élément 200 possède un plateau supérieur 21 muni de dents 26 sur sa face supérieure et d'un coulisseau 23 et d'une rainure (non visible) sur sa face inférieure. La came 30, la vis 40 et l'écrou 50 sont identiques aux éléments correspondants de la forme de réalisation précédente.

## Revendications

1. Implant notamment pour le remplacement d'un corps vertébral en chirurgie du rachis, comprenant des premiers et second éléments d'appui (100, 200; 20a, 20b) contre des plateaux vertébraux sus- et sous-jacents, des moyens (13, 23; 13a, 13b, 23) pour retenir les deux éléments d'appui l'un au-dessus de l'autre et au moins un organe mobile (30; 30a, 30b) apte à faire varier la distance encre les deux éléments d'appui, les moyens de retenue comprenant au moins un moyen à glissière (13, 23; 13a, 13b, 23) prévus entre les deux éléments d'appui, **caractérisé en ce que** le ou chaque organe mobile est constitué par une came à positions discrètes (30; 30a, 30b) apte à être entraînée en rotation autour d'un axe essentiellement horizontal et essentiellement parallèle au plan sagittal.

2. Implant selon la revendication 1, **caractérisé en ce que** la ou chaque came à positions discrètes (30; 30a, 30b) présente en section transversale un contour en forme de polygone irrégulier.

3. Implant selon la revendication 2, **caractérisé en ce que** la ou chaque came (30; 30a, 30b) possède une pluralité de paires de faces (31, 31'; 32, 32'; 33, 33'), les faces d'une même paire étant mutuellement parallèles, et chaque paire de faces possédant une distance entre faces (d1, d2, d3) différente de celle des autres paires.

4. Implant selon la revendication 3, **caractérisé en ce que** la came possède trois paires de faces (31, 31'; 32, 32'; 33, 33').

5. Implant selon l'une des revendications 3 et 4, **caractérisé en ce que** chaque élément d'appui possède un plateau d'appui (11, 21; 21a, 21b) sur une face duquel est ménagée une rainure (12, 22; 12a, 12b, 22), et **en ce qu'**au moins certaines paires de faces (32, 32'; 33, 33') sont dimensionnées de manière à présenter une longueur, selon la circonférence de la came, très légèrement inférieure à la largeur de chaque rainure.

6. Implant selon la revendication 5, **caractérisé en ce que** les faces (32, 32') d'au moins une paire de faces de la came présentent une longueur, selon la circonférence de la came, qui est ajustée à la largeur des rainures (12, 22; 12a, 12b, 22) des plateaux d'appui à l'aide d'un décrochement (321, 321') prévu au niveau de sa transition avec une face voisine (33, 33').

7. Implant selon l'une des revendications 1 à 6, **caractérisé en ce que** la ou chaque came (30; 30a, 30b) est montée à rotation et à translation sur le ou chaque moyen à glissière.

8. Implant selon la revendication 7, **caractérisé en ce que** le ou chaque moyen à glissière comprend un montant creux formant glissière (13; 13a, 13b) formé à partir d'un élément (10; 100) de l'implant et un élément formant coulisseau (23) formé à partir d'un autre élément (200; 20a, 20b) de l'implant, et **en ce que** la glissière et le coulisseau sont traversés par des ouvertures allongées (14, 15, 24; 14a, 15a, 24; 14b, 15b, 24) pour le passage d'un axe (40; 40a, 40b) de montage de la came.

9. Implant selon la revendication 8, **caractérisé en ce que** l'axe de montage de la came est défini par une vis (40; 40a, 40b) traversant un passage central (34) de la came et lesdites ouvertures allongées, et engagée dans un écrou (50; 50a, 50b).

10. Implant selon la revendication 9, **caractérisé en ce que** l'une des ouvertures allongées (14; 14a; 14b) de la glissière est une encoche apte à assurer le blocage de l'écrou (50; 50a, 50b) contre sa rotation.

11. Implant selon l'une des revendications 1 à 10, **caractérisé en.ce que** les éléments d'appui (100, 200; 20a, 20b) possèdent à leur surface extérieure des dents (26) d'ancrage dans des vertèbres sus- et sous-jacente à l'implant.

12. Implant selon l'une des revendications 1 à 11, **caractérisé en ce qu'**il comprend deux éléments d'appui (20a, 20b) et un élément central (10), deux cames (30a, 30b) opérant respectivement entre l'élément d'appui supérieur (20a) et l'élément central (10) et entre l'élément d'appui inférieur (20b) et l'élément central (10), et deux moyens formant glissières (13a, 23; 13b, 23) opérant respectivement entre l'élément d'appui supérieur (20a) et l'élément central (10) et entre l'élément d'appui inférieur (20b) et l'élément central (10).

## Patentansprüche

1. Implantat, welches insbesondere für den Ersatz eines Wirbelkörpers in der Chirurgie der Wirbelsäule verwendet wird, und das erste und zweite Stützelemente (100, 200; 20a, 20b) für die Auflage auf den darunter und darüber liegenden Wirbelflächen enthält, sowie Mittel (13, 23; 13a, 13b, 23), um diese beiden Stützelemente übereinander zu halten, und das mindestens ein bewegliches Organ (30; 30a, 30b) aufweist, mit dessen Hilfe der Abstand zwischen den beiden Stützelemente verändert werden kann, wobei die Stützelemente mindestens eine Gleitvorrichtung (13, 23; 13a, 13b, 23) zwischen den beiden Stützelementen aufweisen,
**dadurch gekennzeichnet, dass**
das oder die beweglichen Organe aus einer Nocke mit diskreten Positionen (30; 30a, 30b) besteht, die um eine Achse gedreht werden kann, welche im wesentlichen horizontal und parallel zu der Krümmungsebene verläuft.

2. Implantat nach Anspruch 1,
**dadurch gekennzeichnet, dass**
die jeweilige(n) Nocke(n) mit diskreten Positionen (30; 30a, 30b) im Querschnitt ein Profil in Form eines unregelmäßigen Polygons besitzt.

3. Implantat nach Anspruch 2,
**dadurch gekennzeichnet, dass**
die Nocke(n) (30; 30a, 30b) eine Vielzahl von Flächenpaaren (31, 31'; 32, 32'; 33, 33') aufweist, wobei die Flächen desselben Paares gegeneinander parallel 30 verlaufen, und dass jedes der Flächenpaare einen Abstand zwischen den Flächen (d1, d2, d3) besitzt, der sich von dem Abstand der anderen Flächen unterscheidet.

4. Implantat nach Anspruch 3,
**dadurch gekennzeichnet, dass**
die Nocke drei Flächenpaare (31, 31'; 32, 32'; 33, 33') besitzt

5. Implantat nach einem der Ansprüche 3 und 4,
**dadurch gekennzeichnet, dass**
jedes der Stützelemente eine Auflageplatte (11, 21; 21a, 21b) enthält, die in einer ihrer Flächen eine Nut (12, 22; 12a, 12b, 22) aufweist, und dadurch, dass mindestens einige der Flächenpaare (32, 32'; 33, 33') so dimensioniert sind, dass sie über den Umfang der Nocke eine Länge haben, die etwas kleiner ist, als die Breite der einzelnen Nuten.

6. Implantat nach Anspruch 5,
**dadurch gekennzeichnet, dass**
die Flächen (32, 32') mindestens eines der Flächenpaare der Nocke über den Umfang der Nocke eine Länge haben, die an die Breite der Nuten (12, 22; 12a, 12b, 22) der Auflageplatten mit Hilfe eines Absatzes (321, 321') angepasst ist, welcher am Übergang zu einer benachbarten Fläche (33, 33') vorgesehen ist.

7. Implantat nach einem der Ansprüche 1 bis 6,
**dadurch gekennzeichnet, dass**
die jeweiligen Nocken (30; 30a, 30b) drehbar und verschiebbar auf den jeweiligen Gleitmitteln angeordnet sind.

8. Implantat nach Anspruch 7,
**dadurch gekennzeichnet, dass**
die jeweiligen Gleitmittel einen hohlen Schenkel aufweisen, der eine Gleitschiene (13, 13a, 13b) bildet und aus einem Element (10; 100) des Implantats besteht, welches einen Schlitten (231) bildet, der aus einem anderen Element (200; 20a, 20b) des Implantats besteht, und dadurch, dass die Gleitschiene und der Schlitten mit länglichen Öffnungen (14, 15, 24; 14a, 15a, 24; 15b, 24) für den Durchgang einer Achse (40; 40a, 40b) für die Montage der Nocke ausgestattet sind.

9. Implantat nach Anspruch 8,
**dadurch gekennzeichnet, dass**
die Montageachse der Nocke durch eine Schraube (40; 40a, 40b) gebildet wird, welche den zentralen Durchgang (34) der Nocke und die länglichen Öffnungen durchquert und in einer Schraubenmutter (50; 50a, 50b) aufgenommen ist.

10. Implantat nach Anspruch 9,
**dadurch gekennzeichnet, dass**
eine der länglichen Öffnungen (14; 14a; 14b) der Gleitschiene aus einer Vertiefung besteht, die eine drehfeste Blockierung der Schraubenmutter (50; 50a, 50b) gewährleisten kann.

11. Implantat nach einem der Ansprüche 1 bis 10,
**dadurch gekennzeichnet, dass**
die Stützelemente (100, 200; 20a, 20b) an ihrer Außenseite Verzahnungen (26) für die Verankerung in den unterhalb und oberhalb des Implantats liegenden Wirbelknochen besitzen.

12. Implantat nach einem der Ansprüche 1 bis 11,
**dadurch gekennzeichnet, dass**
es zwei Stützelemente (20a, 20b) und ein zentrales Element (10) aufweist, sowie zwei Nocken (30a, 30b), die jeweils zwischen dem oberen Stützelement (20a) und einem zentralen Element (10) und zwischen dem unteren Stützelement (20b) und dem zentralen Element (10) wirken, sowie zwei Gleitschienen (13a, 23; 13b, 23) aufweist, die jeweils zwischen dem oberen Stützelement (20a) und dem zentralen Element (10) und dem unteren Stützelement (20b) und dem zentralen Element (10) wirken.

## Claims

1. An implant specifically for replacing a vertebral body in surgery of the spine, the implant comprising first and second bearing elements (100, 200; 20a, 20b) for bearing against under- and overlying vertebral plates, retaining means (13, 23; 13a, 13b, 23) for retaining the two bearing elements one above the other, and at least one moving member (30; 30a, 30b) suitable for varying the distance between said bearing elements, the retaining means comprising at least one slideway means (13, 23; 13a, 13b, 23) provided between the two bearing elements, the implant being **characterized in that** the or each moving member is constituted by a cam (30; 30a, 30b) having discrete positions suitable for being rotated about an axis that is essentially horizontal and essentially parallel to the sagittal plane.

2. An implant according to claim 1, **characterized in that** the or each discrete-position cam (30; 30a, 30b) has, in cross-section, an outline in the form of an irregular polygon.

3. An implant according to claim 2, **characterized in that** the or each cam (30; 30a, 30b) possesses a plurality of pairs of faces (31, 31'; 32, 32'; 33, 33'), the faces in each pair being mutually parallel, and each pair of faces being spaced apart at a distance (d1, d2, d3) different from the spacing between the other pairs of faces.

4. An implant according to claim 3, **characterized in that** the cam has three pairs of faces (31, 31'; 32, 32'; 33, 33').

5. An implant according to claim 3 or 4, **characterized in that** each bearing element possesses a bearing plate (11, 21; 21a, 21b) having a groove (12, 22; 12a, 12b, 22) formed in one face thereof, and **in that** at least some of the pairs of faces (32, 32'; 33, 33') are dimensioned in such a manner as to be of a length in the circumferential direction of the cam that is very slightly shorter than the width of each groove.

6. An implant according to claim 5, **characterized in that** the faces (32, 32') of at least one pair of faces of the cam are of a length, in the circumferential direction of the cam, which is matched to the width of the grooves (12, 22; 12a, 12b, 22) in the bearing plates by means of respective setbacks (321, 321') provided in transitions between said faces and adjacent faces (33, 33').

7. An implant according to any one of claims 1 to 6, **characterized in that** the or each cam (30; 30a, 30b) is mounted to move in rotation and in translation on the or each slideway means.

8. An implant according to claim 7, **characterized in that** the or each slideway comprises a hollow slideway-forming upright (13; 13a, 13b) formed from one element (10; 100) of the implant, and a slider-forming element (23) formed from another element (200; 20a, 20b) of the implant, and **in that** the slideway and the slider have elongate openings (14, 15, 24; 14a, 15a, 24; 14b, 15b, 24) passing through them to pass a cam-mounting shaft (40; 40a, 40b).

9. An implant according to claim 8, **characterized in that** the cam-mounting shaft is defined by a screw (40; 40a, 40b) passing through a central passage (34) of the cam and through said elongate openings, and engaged in a nut (50; 50a, 50b).

10. An implant according to claim 9, **characterized in that** one of the elongate openings (14; 14a; 14b) of the slideway is a notch suitable for preventing the nut (50; 50a, 50b) from rotating.

11. An implant according to any one of claims 1 to 10, **characterized in that** the bearing elements (100, 200; 20a, 20b) have teeth (26) on their outside surfaces for engaging in the vertebrae lying over and under the implant.

12. An implant according to any one of claims 1 to 11, **characterized in that** it comprises two bearing elements (20a, 20b) and a central element (10), two cams (30a, 30b) operating respectively between the top bearing element (20a) and the central element (10) and between the bottom bearing element (20b) and the central element (10), and two slideway-forming means (13a, 23; 13b, 23) operating respectively between the top bearing element (20a) and the central element (10) and between the bottom bearing element (20b) and the central element (10).
